# EUROPEAN PATENT APPLICATION

(11) **EP 1 000 945 A1**
(43) Date of publication of application: **17.05.2000**
(21) Application number: 98924626.9
(22) Date of filing: 16.06.1998
(51) Int. Cl.: C07D 501/04, C07D 499/00, C07D 499/08, C07D 498/04, C07D 463/06

(54) **PROCESS FOR PRODUCING $g(b)-LACTAM DERIVATIVES**

(30) Priority: 24.06.1997 JP 18441497
(71) Applicant: OTSUKA KAGAKU KABUSHIKI KAISHA, Osaka-shi, Osaka-fu 540-0021 (JP)
(72) Inventor: KAMEYAMA, Yutaka, Otsuka Kagaku KK, Tokushima Lab., Tokushima-shi, Tokushima-ken 771-0193 (JP)
(74) Representative: Winter, Brandl, Fürniss, Hübner, Röss, Kaiser, Polte Partnerschaft
(86) International application number: JP9802630
(87) International publication number: WO9858931

(57) **Abstract**

A process for preparing a β-lactam derivative represented by the formula (2)

A-COOH (2)

wherein A is a residue of the β-lactam derivative, the process being characterized in that a β-lactam derivative having a protected carboxyl group which derivative is represented by the formula (1)

A-COO-X (1)

wherein A is as defined above and X is a benzyl group which may have an electron-donating group as a substituent on a phenyl ring or a diphenylmethyl group which may have an electron-donating group as a substituent on a phenyl ring, is treated with aluminum halide in an aliphatic ether solvent to give the derivative of the formula (2); and that the removed group X can be recovered as X-OH for reuse.

## Description

### TECHNICAL FIELD

The present invention relates to a process for preparing a β-lactam derivative. The β-lactam derivatives of the present invention can be used, for example, as intermediates of cefazolin (see Handbook of Latest Antibiotics, 9th ed.) which is useful as a cephalosporin injection.

### BACKGROUND ART

β-Lactam derivatives used as antibiotics generally have a carboxyl group in the molecule and are used in most cases in the form of a free carboxylic acid or a pharmaceutically acceptable salt thereof. However, in the process of preparing β-lactam antibiotics, the carboxyl group is protected with a proper protective group in most cases, and eventually the protective group must be removed in a high yield without destroying other moieties within the molecule.

Processes are heretofore known for preparing a β-lactam derivative represented by the formula (2)

A-COOH (2)

wherein A is a residue of the β-lactam derivative by removing a protective group X of carboxylic acid from a β-lactam derivative having a protected carboxyl group which derivative is represented by the formula (1)

A-COO-X (1)

wherein A is as defined above and X is a benzyl group which has an electron-donating group as a substituent on a phenyl ring or a diphenylmethyl group which has an electron-donating group as a substituent on a phenyl ring. Known processes include, for example, a process wherein the β-lactam derivative of the formula (1) is subjected to catalytic reduction using a precious metal catalyst, and a process wherein the β-lactam derivative of the formula (1) is treated with an acid. The latter process further includes a process using trifluoroacetic acid [J. Am. Chem. Soc., 91, 5674 (1969)], a process using formic acid [Chem. Pharm. Bull., 30, 4545 (1982)], a process wherein the derivative is reacted with aluminum chloride in the presence of anisole [Tetrahedron Lett., 2793 (1979)], a process using a phenol [J. Org. Chem., 56, 3633 (1991)] and so on. However, these conventional processes have the following drawbacks.

The process involving a catalytic reduction using a precious metal catalyst has a drawback that usually a β-lactam antibiotic has a sulfide linkage in the molecule which linkage poisons the catalyst, resulting in a need to use an expensive precious metal in a large amount. Further, the process is not applicable to a β-lactam derivative having, in the same molecule, a group which can be reduced such as a nitro group, carbon-carbon multiple bond or the like. Moreover, the process can not remove, in most cases, such protective groups as a benzyl group which has an electron-donating group as a substituent on a phenyl ring or a diphenylmethyl group which has an electron-donating group as a substituent on a phenyl ring.

The process using an acid such as trifluoroacetic acid poses the following problems. The process usually requires the use of expensive trifluoroacetic acid in a large amount, and would entail a large quantity of loss in recovery or reuse of trifluoroacetic acid after the reaction for the removal of protective group. Furthermore, the process gives a carboxylic acid compound in a low yield because the β-lactam derivative which is unstable against an acid decomposes during recovery. The process using formic acid requires the use of expensive 98-99% formic acid as a solvent in a large excess and gives a carboxylic acid compound in a low yield, as is the case with the reaction with trifluoroacetic acid, due to the decomposition of β-lactam derivative which is unstable against an acid during recovery or reuse. The process using aluminum chloride in the presence of anisole and the process using a phenol are disadvantageous in that the protective group can not be reused because the removed group X is scavenged by anisole or phenol, making it impossible to use an expensive protective group having a substituent. As described above, an efficient, industrial process has not been heretofore developed which is beneficial in that the protective group of carboxylic acid can be removed in a high yield from the β-lactam derivative of the formula (1) having a protected carboxylic group and that the removed protective group can be reused.

An object of the present invention is to provide a novel technique for preparing a β-lactam derivative having a carboxyl group represented by the formula (2) with a high efficiency without use of an expensive reagent, wherein the protective group X of carboxylic acid can be removed from a β-lactam derivative with a protected carboxyl group represented by the formula (1) as an alcohol which can be reused.

Another object of the invention is to provide a process for preparing a β-lactam derivative having a carboxyl group with a high efficiency without a need to use a scavenger for scavenging the removed group such as anisole, phenol or the like such that the removed group can be recovered as an alcohol and can be reused in a reaction for introducing a protective group.

A further object of the invention is to provide a process for preparing a β-lactam derivative having a carboxyl group with a high efficiency, wherein an aliphatic ether solvent is usable which facilitates the recovery and the β-lactam derivative can be easily isolated merely by collecting the solid precipitated from the reaction mixture with a filter on removal of protective group.

### DISCLOSURE OF THE INVENTION

The present invention provides a process for preparing a β-lactam derivative represented by the formula (2)

A-COOH (2)

wherein A is a residue of the β-lactam derivative, the process being characterized in that a β-lactam derivative having a protected carboxyl group which derivative is represented by the formula (1)

A-COO-X (1)

wherein A is as defined above and X is a benzyl group which may have an electron-donating group as a substituent on a phenyl ring or a diphenylmethyl group which may have an electron-donating group as a substituent on a phenyl ring, is treated with aluminum halide in an aliphatic ether solvent to give the derivative of the formula (2); and that the removed group X can be recovered as X-OH for reuse.

According to the present invention, the β-lactam derivative which is unstable against an acid is precipitated from the reaction mixture on removal of protective group, whereby the stability of the β-lactam derivative is assured. In addition, the removed group is recovered in the form of an alcohol and therefore can be reused in a reaction for introducing a protective group. In the process, an ether solvent such as dioxane or THF is usable which means that the solvent can be easily recovered. Since the β-lactam derivative is precipitated from the reaction mixture, the derivative can be easily isolated.

Examples of the residue of the β-lactam derivative represented by A in the specification are the groups represented by the formula (3). 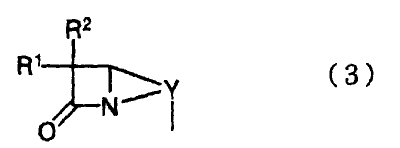 wherein

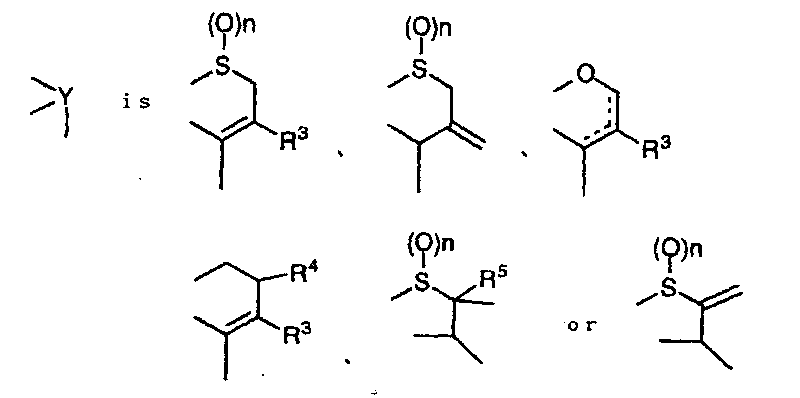

In the formula (3), R¹ is a known substituent in the 7-position of cephalosporin, R² is a usual substituent in the 6-position of penicillin or in the 7-position of cephalosporin, R³ is a known substituent in the 3-position of cephalosporin, R⁴ is a known substituent in the 2-position of 1-carbacephem, R⁵ is a known substituent in the 2-position of penicillin, and n is 0, 1 or 2.

Specific examples of these groups are as follows.

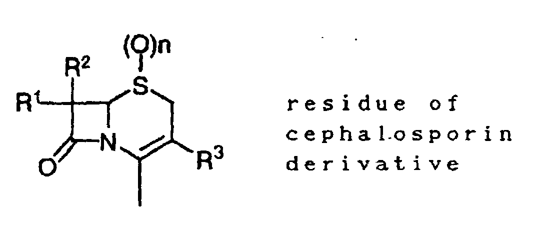

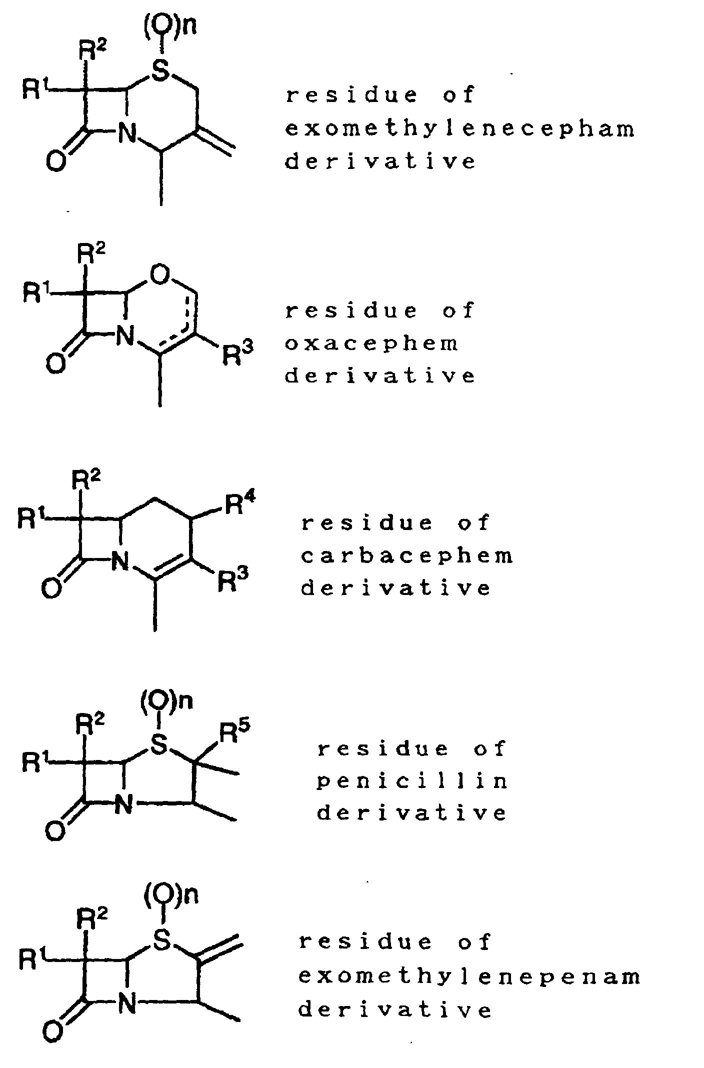

Examples of the groups mentioned in the specification are as follows. Exemplary of the halogen atom are fluorine atom, chlorine atom, bromine atom and iodine atom. Examples of the lower alkyl group are straight-chain or branched-chain C₁-C₄ alkyl groups such as methyl, ethyl, n-propyl, isopropyl, n-butyl, isobutyl, sec-butyl and tert-butyl. Exemplary of the aryl group are phenyl, tolyl, xylyl and naphthyl.

Examples of R¹ groups include known substituents in the 7-position of cephalosporin, more specifically hydrogen atom, methoxy, ethoxy and like lower alkoxy groups and formamido.

Examples of R² groups are known substituents in the 6-position of penicillin or in the 7-position of cephalosporin as disclosed in Mary C. Griffiths, USAN and the USP dictionary of drugs names. More specific examples are hydrogen atom, halogen atom, amino and amido. Examples of the amido group are phenoxyacetamido, p-methylphenoxyacetamido, p-methoxyphenoxyacetamido, p-chlorophenoxyacetamido, p-bromophenoxyacetamido, phenylacetamido, p-methylphenylacetamido, p-methoxyphenylacetamido, p-chlorophenylacetamido, p-bromophenylacetamido, phenylmonochloroacetamido, phenyldichloro-acetamido, phenylhydroxyacetamido, phenylacetoxyacetamido, α-oxophenylacetamido, thienylacetamido, benzamido, p-methylbenzamido, p-t-butylbenzamido, p-methoxybenzamido, p-chlorobenzamido, p-bromobenzamido, the groups described in Theodora W. Greene "Protective Groups in Organic Synthesis, 1981 by John Wiley & Sons, Inc." (hereinafter referred to merely as the "literature"), Chap. 7 (pp.218-287), phenylglycylamido, phenylglycylamido with a protected amino group, p-hydroxyphenylglycylamido and p-hydroxyphenylglycylamido with amino or hydroxyl or both protected. Examples of protective groups for amino groups of phenylglycylamido and p-hydroxyphenylglycylamido are those described in the literature, Chap. 7 (pp.218-287). Examples of protective groups for hydroxyl group of p-hydroxyphenylglycylamido group are those described in the literature, Chap. 2 (pp.10-72).

Examples of R³ groups are known substituents in the 3-position of cephalosporin disclosed in USAN and the USP dictionary of drugs names. More specific examples are hydrogen atom; hydroxy; chlorine, bromine, iodine, fluorine and like halogen atoms; methoxy, ethoxy and like lower alkoxy groups; vinyl, propenyl, 2,2-dibromovinyl and like substituted or unsubstituted alkenyl groups; ethynyl, propargyl and like lower alkynyl groups; methyl, ethyl and like lower alkyl groups; methoxymethyl, ethoxymethyl and like lower alkoxymethyl groups; acetoxymethyl; carbamoyloxymethyl; 1,2,3-triazol-4-ylthiomethyl, 5-methyl-1,3,4-thiadiazol-2-ylthiomethyl, 1-methyltetrazol-5-ylthiomethyl, 1-sulfomethyltetrazol-5-ylthiomethyl, 1-carboxymethyltetrazol-5-ylthiomethyl 1-(2-dimethylaminoethyl)tetrazol-5-ylthiomethyl, 1,3,4-thiadiazol-5-ylthiomethyl, 1-(2-hydroxyethyl)tetrazol-5-ylthiomethyl and like heterocyclic thiomethyl groups; 1-methylpyrrolidinomethyl, pyridiniummethyl and 1,2,3-triazole.

Examples of R⁴ groups are known substituents in the 2-position of 1-carbacephem. More specific examples are hydrogen atom; hydroxy; chlorine, bromine and like halogen atoms; methyl, ethyl and like lower alkyl groups; methoxy, ethoxy and like lower alkoxy groups; formyloxy, acetyloxy, propionyloxy and like lower acyloxy groups; methylthio, ethylthio and like lower alkylthio groups; 1,2,3-triazol-4-ylthio, 5-methyl-1,3,4-thiadiazol-2-ylthio, 1-methyltetrazol-5-ylthio and like heterocyclic thio groups.

Examples of R⁵ groups include all of groups exemplified above as R³ groups. The symbol n is 0, 1 or 2. A broken line in the residue of said oxacephem derivative shows that the carbons in the 2- and 3-positions are in the form of double bond or the carbons in the 3- and 4-positions are in the form of double bond.

Examples of the electron-donating groups substituted on the phenyl ring of the benzyl group or diphenylmethyl group represented by X in the specification are hydroxy; methyl, ethyl, tert-butyl and like lower alkyl groups; and methoxy, ethoxy and like lower alkoxy groups. The diphenylmethyl group includes a type of the group which is a substituted or unsubstituted phenyl group bonded in the molecule via methylene chain or hetero-atom. Exemplary of the benzyl group optionally having an electron-donating group as a substituent on a phenyl ring and the diphenylmethyl group optionally having an electron-donating group as a substituent on a phenyl ring are benzyl, p-methoxybenzyl, diphenylmethyl, 3,4,5-trimethoxybenzyl, 3,5-dimethoxy-4-hydroxybenzyl, 2,4,6-trimethylbenzyl, piperonyl, ditolylmethyl, naphthylmethyl and 9-anthryl.

The β-lactam derivatives of the formula (1) for use in this invention can be any of conventional compounds which fall within a purview of the formula (1).

Aluminum halides usable in the present invention include aluminum chloride, aluminum bromide and aluminum iodide. Among them, aluminum chloride is preferred. Aluminum halide is used in an amount of usually 1 mole per mole of the compound (1). Suitably it is added in an amount ranging from 1 to 20 moles until the treatment of the compound (1) is completed.

Exemplary of the aliphatic ether solvent which can be used in this invention are diethyl ether, diisopropyl ether and like chain dialkyl ethers, dioxane, dioxolane, tetrahydrofuran and like cyclic ethers, and ethylene glycol dimethyl ether, diglyme, triglyme and like ethylene glycol dialkyl ethers. Preferred solvents are dioxane, dioxolane and tetrahydrofuran. These solvents can be used either alone or in combination. The amount of the solvent used is in the range of about 0.5 to about 200 liters, preferably about 1 to about 50 liters, per kilogram of the compound of the formula (1). The reaction is carried out at a temperature in the range of -10 to 80°C, preferably 0 to 50°C.

The compound of the formula (2) can be obtained as a substantially pure product by collecting the solid precipitated from the reaction mixture with a filter or by usual extraction or recrystallization after completion of the reaction. The reaction product can be purified, of course, by other methods. Usual extraction can be conducted, for example, by adding sodium hydrogencarbonate or sodium carbonate and a hydrophobic organic solvent and extracting the β-lactam derivative of the formula (2) in the aqueous layer, followed by a usual procedure, whereby the compound (2) is obtained in a high yield. Examples of useful hydrophobic organic solvents are ethyl acetate, butyl acetate and like esters, methylene chloride, chloroform and like halogenated hydrocarbons. The removed group X can be recovered as a re-usable alcohol (X-OH) by filtering the reaction mixture with the deposit separated out therein or by usual extraction, followed by concentration or distillation of the filtrate or the hydrophobic organic solvent layer or recrystallization therefrom.

### BEST MODE OF CARRYING OUT THE INVENTION

The present invention will be described in detail with reference to the following Example, Comparative Example and Reference Example.

### Example 1

There were weighed out 200 mg of the compound represented by the formula (1) (A= residue of cephalosporin derivative, X= p-methoxybenzyl, R¹= hydrogen atom, R²= phenylacetamido, R³=5-methyl-1,3,4-thiadiazol-2-ylthiomethyl, and n=0, i.e. compound 1a) and 200 mg of aluminum chloride. Then 4 ml of dioxane was added. The resulting solution was stirred at room temperature for 1.5 hours. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2a (151 mg, 95%). ¹H NMR(Acetone-d₆) d 2.70(s, 3H), 3.65(s, 2H), 3.75(bs, 2H), 4.35(d, J=14Hz, 1H), 4.53(d, J=14Hz, 1H), 5.08(d, J=5Hz, 1H), 5.78(dd, J=5, 9Hz, 1H), 7.20∼7.45(m, 5H), 7.87(d, J=9H, 1H).

### Reference Example 1 (Recovery of protective group X)

The ethyl acetate solution (the solution containing the neutral component) obtained by extraction with the ethyl acetate-sodium hydrogencarbonate aqueous solution in Example 1 was concentrated under reduced pressure. The obtained residue was purified by a silica gel column chromatography (benzene/ethyl acetate=4/1), giving p-methoxybenzyl group as a protective group in the form of p-methoxybenzyl alcohol (42 mg, 90%).
¹H NMR(DMSO-d₆) d 3.72(s, H), 4.40(d, J=6Hz, 2H), 5.02(t, J=6Hz, 1H), 6.87(d, J=8Hz, 2H), 7.21(d, J=8Hz, 2H).

The obtained product is easily converted into p-methoxybenzyl chloride merely by treatment with concentrated hydrochloric acid, and it can be reused as a protective group-introducing agent.

### Comparative Example 1 (Recovery of protective group X by anisole/aluminum chloride method)

A 200 mg quantity of the compound 1a represented by the formula (1) was dissolved in 2 ml of nitromethane. Then 75 ml of anisole was added to the solution. The mixture was cooled to 3°C. Separately, 200 mg of aluminum chloride was weighed out and was dissolved in 2 ml of nitromethane, followed by cooling to 3°C. The nitromethane solution of the compound (1a) and anisole was added dropwise by a bridge to the nitromethane solution of aluminum chloride. A reaction was continued at this temperature for 2 hours, and the same subsequent procedure as in Example 1 was effected, giving the compound 2a (145 mg, 91%).

The obtained ethyl acetate solution containing the neutral component and the basic component was concentrated, and the concentrate was purified by column chromatography, giving a compound having a p-methoxybenzyl group coupled with anisole (CH₃O-C₆H₄-CH₂C₆H₄-OCH₃, o,p-mixture) (65 mg, 80%).

### Examples 2 to 4

The reactions were conducted in the same manner as in Example 1 except that the amount of aluminum chloride and reaction time were changed as follows.

**Table 1**

| Ex. | aluminum chloride (mg) | reaction time (h) | yield (%) |
|---|---|---|---|
| 2 | 50 | 9 | 82 |
| 3 | 100 | 4 | 84 |
| 4 | 300 | 1.5 | 92 |

### Examples 5 to 7

The reactions were conducted in the same manner as in Example 1 except that the solvent and reaction time were changed as follows.

**Table 2**

| Ex. | Solvent | reaction time (h) | yield (%) |
|---|---|---|---|
| 5 | THF | 3 | 80 |
| 6 | dioxolane | 6 | 87 |
| 7 | dimethoxyethane | 5 | 75 |

### Example 8

There were weighed out 200 mg of the compound represented by the formula (1) (A= residue of cephalosporin derivative, X= p-methoxybenzyl, R¹= hydrogen atom, R²= phenylacetamido, R³=1-methyltetrazol-5-ylthiomethyl, and n=0, i.e. compound 1b) and 200 mg of aluminum chloride. Then 4 ml of dioxane was added. The resulting solution was stirred at room temperature for 1 hour. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2b (142 mg, 90%).
¹H NMR(Acetone-d₆) d 3.65(s, 2H), 3.75(s, 2H), 3.98(s, 3H), 4.37(s, 2H), 5.06(d, J=5Hz, 1H), 5.74(dd, J=5, 9Hz, 1H), 7.27∼ 7.40(m, 5H), 7.90(d, J=9Hz, 1H).

### Example 9

There were weighed out 200 mg of the compound represented by the formula (1) (A= residue of cephalosporin derivative, X= p-methoxybenzyl, R¹= hydrogen atom, R²= phenylacetamido, R³=acetoxymethyl, and n=0, i.e. compound 1c) and 200 mg of aluminum chloride. Then 4 ml of dioxane was added. The resulting solution was stirred at room temperature for 2 hours. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2c (147 mg, 96%).
¹H NMR(Acetone-d₆) d 2.04(s, 3H),3.52(d, J=17Hz, 1H), 3.61(d, J=17Hz, 1H), 3.67(s, 2H), 4.86(d, J=14Hz, 1H), 5.04(d, J=14Hz,1H), 5.08(d, J=5Hz, 1H), 5.35∼5.60(bs, 3H), 5.80(dd, J=5,8Hz, 1H),7.27∼7.45(m, 5H), 7.87(d, J=8Hz, 1H).

### Example 10

There were weighed out 200 mg of the compound represented by the formula (1) (A= residue of penicillin derivative, X= diphenylmethyl, R¹= hydrogen atom, R²= hydrogen atom, R⁵=1,2,3-triazol-1-yl, and n=2, i.e. compound 1d) and 200 mg of aluminum chloride. Then 4 ml of dioxane was added. The resulting solution was stirred at room temperature for 1 hour. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2d (118 mg, 92%).
¹H NMR(DMSO-d₆) d 1.31(s, 3H),3.29(dd, J=1, 16Hz, 1H), 3.68(dd, J=4, 16Hz, 1H), 4.76(s, 1H),4.89(d, J=15Hz, 1H),5.16(dd, J=1, 4Hz, 1H), 5.22(d, J=15Hz, 1H),7.76(d, J=1Hz, 1H), 8.07(d, J=1Hz, 1H).

### Reference Example 2 (Recovery of protective group X)

The ethyl acetate solution (the solution containing the neutral component) obtained by extraction with the ethyl acetate-sodium hydrogencarbonate aqueous solution in Example 10 was concentrated under reduced pressure. The obtained residue was purified by a silica gel column chromatography (benzene/ethyl acetate=5/1), giving diphenylmethyl group as a protective group in the form of benzhydrol (72 mg, 92%).
¹H NMR(DMSO-d₆) δ5.67(d, J=4Hz, 1H), 5.87(d, J=4Hz, 1H), 7.14 ∼7.38(m, 10H)

### Example 11

There were weighed out 200 mg of the compound represented by the formula (1) (A= residue of cephalosporin derivative, X= diphenylmethyl, R¹= hydrogen atom, R²= phenylacetamido, R³= chlorine atom, and n=0, i.e. compound 1e) and 200 mg of aluminum chloride. Then 4 ml of dioxane was added. The resulting solution was stirred at room temperature for 2 hours. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2e (122 mg, 90%).
¹H NMR(DMSO-d₆) d 3.47(d, J=14Hz, 1H),3.55(d, J=14Hz, 1H), 3.68(d, J=18Hz, 1H), 3.95(d, J=18Hz, 1H), 5.15(d, J=5Hz, 1H), 5.68(dd, J=5, 8Hz, 1H), 7.18∼7.32(m, 5H), 9.17(d, J=8Hz, 1H)

### Example 12

There were weighed out 200 mg of the compound represented by the formula (1) (A= residue of cephalosporin derivative, X= p-methoxybenzyl, R¹= hydrogen atom, R²= phenylacetamido, R³=chlorine atom, and n=0, i.e. compound 1f) and 200 mg of aluminum chloride. Then 4 ml of dioxane was added. The resulting solution was stirred at room temperature for 2 hours. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2e (131 mg, 88%). The obtained carboxylic acid 2e was completely identical with that of Example 11 in ¹H NMR.

### Example 13

There were weighed out 200 mg of the compound represented by the formula (1) (A= residue of exomethylenecepham derivative, X= p-methoxybenzyl, R¹= hydrogen atom, R²= phenylacetamido, and n=0, i.e. compound 1g) and 200 mg of aluminum chloride. Then 4 ml of dioxane was added. The resulting solution was stirred at room temperature for 2 hours. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2g (128 mg, 87%).
¹H NMR(Acetone-d₆) d 3.35(d, J=14Hz, 1H), 3.69(d, J=14Hz, 1H), 3.65(s, 2H), 5.08(s, 1H), 5.28(s, 2H), 5.34(d, J=5Hz, 1H), 5.57(dd, J=5, 9Hz, 1H), 7.05∼7.45(m, 5H),7.75(d, J=9Hz, 1H)

### Example 14

There were weighed out 200 mg of the compound represented by the formula (1) (A= residue of exomethylenecepham derivative, X= diphenylmethyl, R¹= hydrogen atom, R²= phenylacetamido, and n=0, i.e. compound 1h) and 200 mg of aluminum chloride. Then 4 ml of dioxane was added. The resulting solution was stirred at room temperature for 1.5 hours. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2g (119 mg, 89%). The obtained carboxylic acid 2g was completely identical with that of Example 13 in ¹H NMR.

### Example 15

There were weighed out 200 mg of the compound represented by the formula (1) (A= residue of exomethylenecepham derivative, X= p-methoxybenzyl, R¹= hydrogen atom, R²= phenoxyacetamido, and n=1, i.e. compound 1i) and 200 mg of aluminum chloride. Then 4 ml of dioxane was added. The resulting solution was stirred at room temperature for 3 hours. The reaction mixture was extracted with an aqueous solution of ethyl acetate-sodium hydrogencarbonate, and the organic layer was extracted again with an aqueous solution of sodium hydrogencarbonate. A mixture of the obtained aqueous solutions was adjusted to a pH of 1-2 with 2N hydrochloric acid, followed by extraction twice with ethyl acetate. The ethyl acetate solution was concentrated under reduced pressure, giving the desired carboxylic acid 2i (128 mg, 85%).
¹H NMR(Acetone-d₆) d 3.80(s, 2H),4.53(s, 2H), 5.05(s, 1H), 5.10(d, J=5Hz, 1H), 5.40(s, 1H), 5.70(s, 1H), 5.90(dd, J=5, 9Hz, 1H), 6.85∼7.50(m, 5H), 9.65(d, J=9Hz, 1H)

### Reference Example 3 (Preparation of cefazolin)

The carboxylic acid 2a obtained in Example 1 can be converted to cefazolin by, for example, the following procedure.

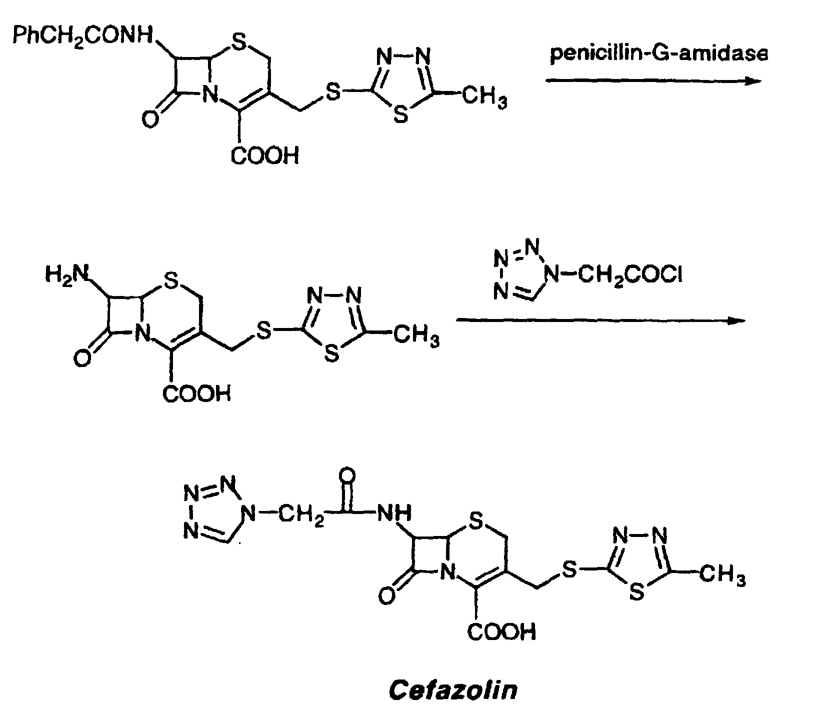

### INDUSTRIAL APPLICABILITY

According to the process of the present invention, the protective group can be removed by a simplified procedure from a β-lactam derivative (1) having a protected carboxyl group. The process of the present invention does not necessitate an expensive reagent required in conventional processes nor a scavenger for scavenging a removed group such as anisole or phenol so that the removed group can be recovered as an alcohol, and a protective group-introducing agent can be recovered in a re-usable form.

An ether solvent which is used in the process of the invention can be easily recovered. Moreover, the β-lactam derivative can be isolated merely by filtering the reaction mixture in which the derivative is separated out on removal of protective group. The process of the invention assures a high stability under the acidic conditions, resulting in the production of the â-lactam derivative (2) with a high purity and a high yield.

## Claims

1. A process for preparing a β-lactam derivative represented by the formula (2)
A-COOH (2)
wherein A is a residue of the β-lactam derivative, the process being characterized in that a β-lactam derivative having a protected carboxyl group which derivative is represented by the formula (1)
A-COO-X (1)
wherein A is as defined above and X is a benzyl group which has an electron-donating group as a substituent on a phenyl ring or a diphenylmethyl group which may have an electron-donating group as a substituent on a phenyl ring, is treated with aluminum halide in an aliphatic ether solvent to give the derivative of the formula (2); and that the removed group X can be recovered as X-OH for reuse.

2. A process as defined in claim 1 wherein the residue of the β-lactam derivative is the group represented by the formula (3) 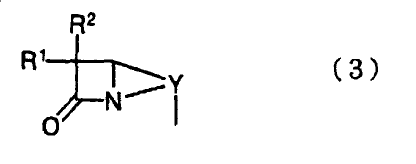 wherein 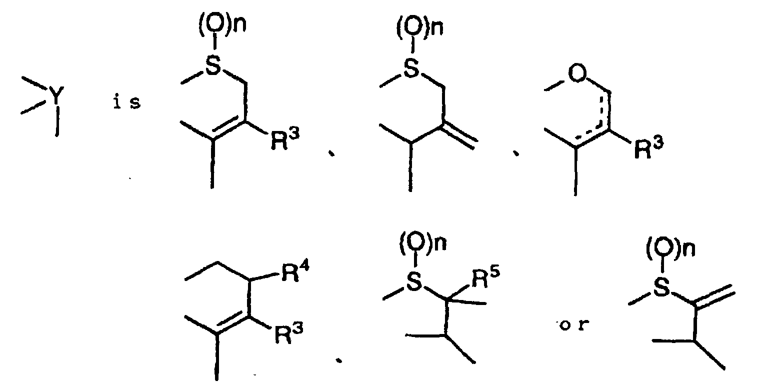
R¹ is a known substituent in the 7-position of cephalosporin, R² is a usual substituent in the 6-position of penicillin or in the 7-position of cephalosporin, R³ is a known substituent in the 3-position of cephalosporin, R⁴ is a known substituent in the 2-position of 1-carbacephem, R⁵ is a known substituent in the 2-position of penicillin, and n is 0, 1 or 2.

3. A process as defined in claim 2 wherein the A is one of the following residues 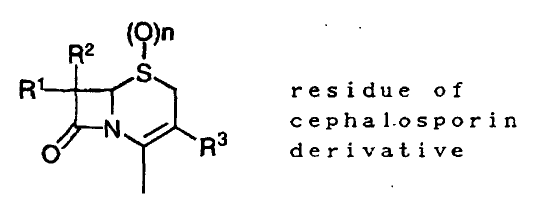 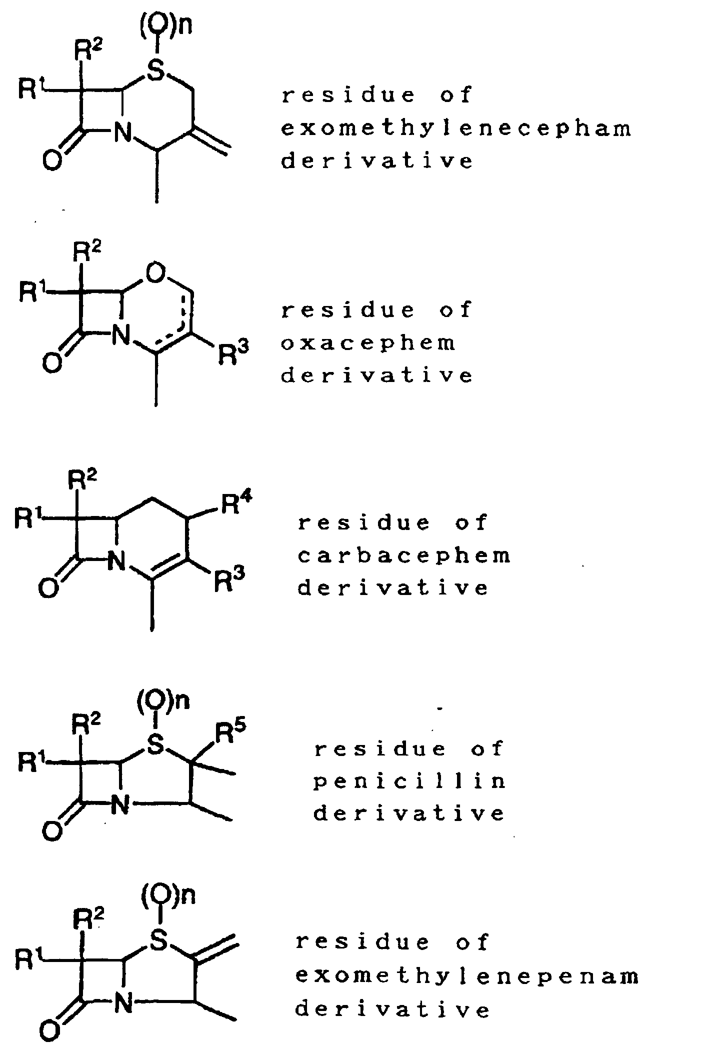
R¹ is a known substituent in the 7-position of cephalosporin, R² is a usual substituent in the 6-position of penicillin or in the 7-position of cephalosporin, R³ is a known substituent in the 3-position of cephalosporin, R⁴ is a known substituent in the 2-position of 1-carbacephem, R⁵ is a known substituent in the 2-position of penicillin, and n is 0, 1 or 2.

4. A process as defined in claim 3 wherein R¹ is hydrogen atom, lower alkoxy group or formamido group, R² is hydrogen atom, halogen atom, amino or amido group, R³ and R⁵ are each hydrogen atom, hydroxy, halogen atom, lower alkoxy group, lower alkenyl group, ethynyl, lower alkyl group, lower alkoxymethyl group, acetoxymethyl, carbamoyloxymethyl, heterocyclic thiomethyl group, 1-methylpyrrolidinomethyl, pyridiniummethyl or 1,2,3-triazole group, R⁴ is hydrogen atom, hydroxy, halogen atom, lower alkyl group, lower alkoxy group, lower acyloxy group, lower alkylthio group or heterocyclic thio group.

5. A process as defined in claim 1 wherein the electron-donating group substituted on the phenyl ring is hydroxy, lower alkyl group or lower alkoxy group.

6. A process as defined in claim 1 wherein the diphenylmethyl group is a type of the group which is a substituted or unsubstituted phenyl group bonded in the molecule via methylene chain or hetero-atom.

7. A process as defined in claim 1 wherein the benzyl group optionally having an electron-donating group as a substituent on a phenyl ring or the diphenylmethyl group optionally having an electron-donating group as a substituent on a phenyl ring is benzyl, p-methoxybenzyl, diphenylmethyl, 3,4,5-trimethoxybenzyl, 3,5-dimethoxy-4-hydroxybenzyl, 2,4,6-trimethylbenzyl, piperonyl, ditolylmethyl, naphthylmethyl or 9-anthryl group.

8. A process as defined in claim 1 wherein the aliphatic ether solvent is chain dialkyl ether, cyclic ether or ethylene glycol dialkyl ether.

9. A process as defined in claim 1 wherein the aluminum halide is aluminum chloride, aluminum bromide or aluminum iodide.
